Europäisches Patentamt

European Patent Office

Office européen des brevets

(11) Publication number: **0 334 383**
**A2**

(12) # EUROPEAN PATENT APPLICATION

(21) Application number: **89105307.6**

(22) Date of filing: **23.03.89**

(51) Int. Cl.⁴: **C12N 15/00 , A01H 3/00**

(30) Priority: **25.03.88 DE 3810286**

(43) Date of publication of application:
**27.09.89 Bulletin 89/39**

(84) Designated Contracting States:
**ES GR**

(71) Applicant: **Max-Planck-Gesellschaft zur Förderung der Wissenschaften e.V.**
**Bunsenstrasse 10**
**D-3400 Göttingen(DE)**

(72) Inventor: **Spena, Angelo, Dr.**
**Sudetenweg 48**
**D-5000 Köln 40(DE)**
Inventor: **Schmülling, Thomas, Dipl.-Biol.**
**Aachener Strasse 17**
**D-5000 Köln 1(DE)**
Inventor: **Salamini, Francesco, Prof. Dr.**
**Goldammer Weg 1**
**D-5000 Köln 30(DE)**
Inventor: **Schell, Joseph St., Prof. Dr.**
**Egelspfad**
**D-5000 Köln 30(DE)**

(74) Representative: **Vossius & Partner**
**Siebertstrasse 4 P.O. Box 86 07 67**
**D-8000 München 86(DE)**

(54) **Transgenic plant with modified physiology, morphology and modified hormone metabolism, tissue cultures of this plant and process for its preparation.**

(57) Transgenic plants with modified physiology, morphology and modified hormone metabolism are described which contain in their genome

a) the coding region of the rolA gene, rolB gene, rolC gene, rolA and rolC gene, rolB gene and rolC gene or rolA and rolB gene of the T_L-DNA of the Ri-plasmid from Agrobacterium rhizogenes and/or the coding region of the IPT (isopentenyladenosine transferase) gene of the T-DNA of Ti-plasmids from Agrobacterium tumefaciens, said coding region(s) being under the control of its/their natural or a heterologous promotor(s), except for the coding region of the rolC gene, which, if it is not contained in combination with one of the other coding regions in the genome, is under the control of a heterologous promotor; and

b) optionally also a selectable marker gene; wherein the coding regions and the marker gene are expressed. Furthermore, tissue cultures are described which contain cells or organs of the transgenic plants and processes for preparing the transgenic plants.

Fig. 1

## Transgenic plant with modified physiology, morphology and modified hormone metabolism, tissue cultures of this plant and process for its preparation

The invention relates to transgenic plants or transgenic organs of plants with modified physiology, morphology and modified hormone metabolism, a process for preparing these plants and organs and tissue cultures containing cells or organs of these plants or cells of the plant organs. The invention also relates to the use of the transgenic plants for breeding plants with modified biochemical, physiological, developmental physiological and morphological feature and the use of the transgenic plant or organ or tissue cultures thereof for preparing secondary plant products.

In many cases of bacteria-induced phytopathogenesis, the hormone biosynthesis plays an important role in the interaction between plant and bacteria. An example is Agrobacterium tumefaciens, the ethiological agent of the crown gall disease. This plant disease is caused by a modified hormone biosynthesis in the infected plant cells. The plant cells are transformed during the infection process. This is provoked by the insertion of a DNA fragment, the "T-DNA", into the genome of the plant cell and subsequent expression of the genetic information contained in the T-DNA. In this way, the hormone metabolism of the plant cell is modified and uncontrolled tumor growth is induced.

Also in the pathogenesis of the hr ("hairy-root") disease, the first event is a transformation of individual cells of the plant. The hr disease is caused by DNA fragments of the Ri-plasmids of Agrobacterium rhizogenes being integrated into the genome of the affected plant cell. The crown galls caused by Agrobacterium tumefaciens are chimaeric tissues as the biosynthesis of auxins (IAA) and cytokinins (IPT) produced by the T-DNA gene products promotes the growth of both transformed and untransformed neighbouring cells. By contrast, the roots induced by Agrobacterium rhizogenes at the site of infection consist only of transformed cells. Accordingly, the products of the rol genes contained in the DNA of the Ri-plasmid and integrated into the plant genome were thought to act mainly, if not exclusively in the transformed cells. Thus it was assumed that these genes do not directly participate in the synthesis of transportable growth factors.

Four rol genes are known, i.e the rolA, rolB, rolC and rolD gene. The rolA, rolB and rolC loci or genes correspond to the open reading frames 10, 11 and 12 of the $T_L$-DNA of the Ri-plasmid. A more detailed description of the loci is given in Spena et al., EMBO J. 6 (1987), p. 3891. The sequence of the $T_L$-DNA of Agrobacterium rhizogenes has been published by Slightom et al., J. Biol. Chem., 261 (1986), p. 108. The binary vector pPCV002 has been described by Koncz and Schell in Mol. Gen. Genet. 204 (1986), p. 383.

The hr syndrome is found in plants which have been regenerated from Ri-transformed roots. Characteristic features of this syndrome are adventitious root formation, high growth rate of root explants in hormone free culture media, reduced apical dominance both in the stems and in the roots, modified leaf and flower morphology, plagiotropic root growth (i.e. with altered geotropism) and reduced fertility. The formation of the fully developed hr syndrome is correlated with the expression of the rolA-, rolB and rolC loci or genes, the gene products of which have a synergistic activity in inducing root formation; Spena et al., loc. cit.

Spena et al., however, merely describe the effect which all three loci, individual ones or subcombinations of the loci, have on the induction of the root formation at the site of infection of infected kalanchoe or tobacco plants. Spena et al. have not, however, described special biological effects of the rolA, rolB, or rolC locus or specific combinations of these loci on whole transgenic plants in which each cell and not only the cell present at the site of infection contains these loci or combinations thereof. The effects of the modification of promotors when using individual rol genes or a combination of more than one rol gene have not been investigated in whole transgenic plants.

Oono et al, Jpn. J. Genet. 62 (1987), 501, disclose transgenic tobacco plants which contain a rolC gene under the control of its natural promotor and therefore show a reduced height in comparison to the wild type plants.

In the field of ornamental plants, there is a great demand for plants with pronounced dwarf growth, plants whose blooming period can be controlled and plants which last long even when kept in flower vases, that is to say take longer to show senescence. Moreover there is a need for plants with an improved root system which grow well even under difficult growing site conditions. Such physiological and morphological properties have so far been achieved only by treating plants with chemicals.

Plants with a marked dwarf growth are particularly of interest in green house cultivation. Since then, the method to achieve such plant morphologies essentially consisted in physically impairing the plant growth, apart from using chemical active ingredients.

Plants which are suitable for breeding plants with the mentioned desired properties have so far been available only to a limited degree.

In the selection of newly cultured plants, a special problem may arise from the fertility of the two participants in cross breeding.

Furthermore, plant tissue cultures are used for the preparation of secondary plant products. However, the yields achieved thereby are not satisfactory.

Consequently, the problem underlying the present invention is to provide plants and plant organs which have modified physiological and morphological properties and lend themselves to the breeding of new plants and to the preparation of advantageous tissue cultures. Furthermore, the technical problem underlying the invention is to provide processes for preparing such plants and organs.

The above-mentioned problem is solved by the embodiments characterized in the patent claims.

Consequently, the invention relates to transgenic plants and plant organs with modified physiology, morphology and modified hormone metabolism, which contain in their genome

a) the coding region of the rolA gene, rolB gene, rolC gene, rolA and rolC gene, rolB and rolC gene or the rolA and rolB gene of the $T_L$-DNA of the Ri-plasmid from Agrobacterium rhizogenes and/or the coding region of the IPT (isopentenyladenosine transferase)-gene of the T-DNA of the Ti-plasmid from Agrobacterium tumefaciens, said coding region(s) being under the control of its/their natural or a heterologous promotor(s), except for the coding region of the rolC gene, which, if it is not contained in combination with another coding region in the genome, is under the control of a heterologous promotor; and

b) optionally also a selectable marker gene; wherein the coding regions and the marker gene are expressed.

The invention essentially stems from the finding of new effects of the rolA, rolB and rolC genes in transgenic plants and organs. These effects include the following:

a) the separate expression of the rol genes in transgenic plants and organs gives rise to specific and distinguishable morphological changes which suggest that these genes have different biological or biochemical targets;

b) the expression of chimaeric genes in which the expression of the coding region of the rolB or rolC gene is controlled by the CaMV ("Cauliflower Mosaic Virus") 35S-promotor, gives rise to new morphological alterations in the transgenic plants and organs;

c) rol gene induced roots are always transformed; and

d) best root growth in in-vitro culture is achieved by combining the rolC gene with the rolB gene or with the rolA gene or by increased expression of the rolC gene.

The results of the present invention show that the rol gene products either have different biochemical targets or have a different influence on the same target playing a role in the control of the plant and organ development. The results further show that the control of the expression of these genes plays a crucial role in the genesis of morphological alterations in the transgenic plants and organs. Each of these three rol genes is by itself capable of eliciting root formation in, for instance, tobacco. A higher number of roots and increased root growth is, however, achieved by the activity of more than one single rol gene.

The plants of the invention do not display the complete hr syndrome which is caused by the combined expression of the coding regions of the rolA, rolB and rolC gene. They only have selected, desired features and by modification in the gene regulation they show new and/or enhanced phenotypical features.

The transgenic plants of the invention have a markedly dwarf growth, a changed blooming period, delayed senescence, a modified root system with, for instance, plagiotropic root growth (growing closely below the surface). They show alterations in their fertility, in the number of their flowers, and/or size of their flowers. To achieve these properties of the transgenic plants the above-mentioned special combinations of the coding regions of the rolA, rolB or rolC genes are used. Moreover, the transgenic plants of the invention can be prepared alone or in combination with one of the rol genes using the coding region of the IPT gene.

The organs of such plants which can either be derived from them or be separately produced have properties corresponding to said plants.

The plants of the invention with increased and/or altered root growth have a selection advantage over the many naturally occurring plants under difficult growth conditions found in nature.

In a preferred embodiment, the heterologous promotor is the HSP ("Heat Shock Promotor")-70 promotor of Drosophila melanogaster.

The HSP-70-promotor of Drosophila melanogaster is described in Spena et al., EMBO J. 4 (1985), p. 2739.

In another preferred embodiment of the invention, the heterologous promotor is a strong constitutive promotor.

In another especially preferred embodiment of the invention the strong constitutive promotor is the CaMV35S-promotor.

In another especially preferred embodiment of the invention. the transgenic plant or organ contains in its genome the coding region of the rolC gene of the $T_L$-DNA of the Ri-plasmid from Agrobacterium rhizogenes, which is under the control of a strong constitutive promotor, preferably under the control of the CaMV35S promotor. If the CaMV35S promotor is used for controlling the rolC gene, the corresponding transgenic plants are dominant male sterile, female fertile and show a prolonged life span or delayed senescence.

By means of a dominant gene conferring male sterility to plants, hybrid seeds can be produced more economically. Even if the female fertility may be slightly reduced, the plant breeder is in a position to prepare a quantity of hybrid seeds sufficient to determine the properties of the hybrid population. The essential advantage of dominant male sterile plants is that crosses between different varieties no longer require emasculation of the recipient. Moreover, if the transgenic plants of the present invention contain a herbicide-ressistant gene, it is possible to cultivate a pure population of male sterile parent plants, because of the combined hereditary transmission of the male sterility and herbicide resistance, the male fertile offspring can be eliminated from the progeny of crosses between male sterile and male fertile plants by treatment with herbicides.

An example of a dominant male sterile transgenic plant according to the invention is a tobacco plant which contains the chimaericCaMV35S-rolC gene.

This plant of the invention shows a markedly dwarf growth and reduced apical dominance. It also has a prolonged life span and a prolongend blooming and budding period or delayed senescence. Its flowers are smaller than those of the wild type plant. Advantageously, these features are passed onto the next plant generation. The pollination of the transgenic tobacco plant of the invention CaMV-C1-I having the aforementioned properties with a wild type plant (SR1), resulted in 23 plants with the transgenic phenotype and 62 plants with the wild type phenotype. In another experiment, the percentage of the plants with a transgenic phenotype was 50%.

To eliminate male sterility or reduced female fertility from the progeny of the afore-mentioned plants which contain and express the CaMV-C gene of the invention, these transgenic CaMV-C plants can be crossed with male fertile plants which are homozygous in respect of one of the following genes:

a) a gene which under the expression control of the CaMV35S promotor or other promotors expresses the antisense-RNA strand of the rolC transcript. On account of the formation of RNA/RNA hybrids, the characteristic phenotype of CaMV-C transgenic plants is not found in the progeny (F1 hybrids); see A. van der Krol, J.N.M. Mol. and A.R. Stuitje, Gene 72 (1988), 45-50;

b) a gene which under the expression control of the CaMV35S promotor or other promotors expresses a ribozyme which catalytically cleaves and thus inactivates the mRNA of the rolC gene in the offspring; see J. Haseloff and W.L. Gerlach, Nature 334, (1988) 585-591.

In another preferred embodiment of the present invention the transgenic plant contains the coding region of the IPT gene under the control of the HSP-70 promotor of Drosophila melanogaster.

The expression of coding regions under the control of a 457 bp DNA fragment of the HSP-70 gene of Drosophila melanogaster which contains the promotor and 199 bp of the untranslated leader sequence can be heat-regulated in tobacco plants; see Spena et al., EMBO J. 4 (1985), p. 2739 and Spena and Schell, Mol. Gen. Genet. 206 (1987) p. 436. This promotor shows a very low basal level of transciption at normal growth temperature, which however can be raised dramatically by activation by a heat shock.

Cytokinins are plant hormones which control various biological phenomena, such as senescence, flower morphology, apical dominance and the formation of adventitious roots. The IPT gene of the T-DNA of the Ti-plasmid of Agrobacterium tumefaciens encodes the isopentenyltransferase. This enzyme catalyzes in the tissue of transgenic plants the rate limiting step in the synthesis of isopentenyladenosine, a cytokinin. Transgenic plants which contain in their genome the IPT gene under the control of its own promotor or of other promotors do not form roots because of the inhibitory effect of the increased cytokinin level.

In order to avoid this problem, the coding region of the IPT gene of the T-DNA of the octopine Ti-plasmid pTi15955 was placed under the control of the HSP-70 promotor of Drosophila melanogaster. The octopine Ti-plasmid pTi15955 is described in Barker et al., Plant Mol. Biol. 2 (1983), p. 325. The chimaericHSIPT gene used consists (1) of the promotor region of the HSP-70 gene of Drosophila (Spena et al., EMBO J. 4 (1985) p. 2739, (2) an untranslated signal region which is formed by the ligation of 199 bp of the untranslated signal region of the HSP-70 gene of Drosophila with 16 bp which are located immediately upstream the ATG start codon of the coding region of the IPT-gene, (3) 702 bp of the coding region of the IPT gene, and (4) 373 bp of the 3'-flank of the IPT gene which extends up to a RsaI restriction cleavage site.

Transgenic tobacco plants which contain said chimaeric gene form roots and have a modified flower morphology. The flowers are bigger and have larger stigmas than wild type plants (SR1). The chimaeric HSIPT gene in these transgenic plants can be induced by a heat-shock regime.

In another preferred embodiment, the transgenic plants or organs co-tain in their genome the coding region of the rolB gene and the rolC gene under the control of a root-specific promotor and thus show increased root formation and modified or advantageous root growth in the absence of other features typical of transgenic rolBC plants or organs.

Transgenic plants or organs with modified physiology, morphology and modified hormone metabolism, which contain in their genome the coding region of the rolA, rolB and rolC gene, are also preferred in accordance with the invention. However at least one of the coding regions is under the control of a heterologous promotor and the other coding regions are optionally under the control of their natural promotors. Preferably, the heterologous promotor is a strong constitutive promotor. The CaMV35S-promotor is especially preferred.

The transgenic plants or organs of the invention are preferably derived from solanaceae, apocynaceae, chenopodiaceae, polygonaceae, boraginaceae, compositae, rubiaceae, scrophulariaceae, caprifoliaceae, leguminosae, araceae, moraceae, asplenium, euphorbiaceae, cabbage types, sunflowers, carrots, paprika (Capsicum annuum), leek, raphanus types, lettuce types, celery (Apium graveolens), onions, fennel (Foeniculum vulgare), wheat, rye, barley, corn (maize), sugar beet, soybean, oats, rice types, dahlias, ornamental tobacco, pelargonia, petunia, primulae, violets, asters, amaranthus types, windflowers, lionsmouths (Antirrhinum), aquilegia, ornamental asparagus, begonias, calceolaria, coleus, chrysanthema, cyclamen, delphinium, carnations, gerbera, impatiens, lupine, gilliflower, portulaceae, crowflower (Ranunculus), sage, gloxinia, tagetes, mullein, zinnia, rosaceae, saponoria, or Panax ginseng.
Plants derived from potatoes or tobacco are especially preferred.
The essential point here is that both dicotyledons and monocotyledons are suitable starting materials for the preparation of the plants of the invention.

Moreover, tissue cultures which contain cells or organs of the above-described transgenic plants or cells of the above-described transgenic plant organs are also provided in accordance with the invention.
In vitro root cultures of transgenic plants which carry the coding region of the rolC gene under the control of the CaMV35S promotor grow at least 10 times faster than the in-vitro root cultures of wild type plants. Further increase in the growth rate can be achieved by combining the rolC gene under the control of the CaMV35S promotor with the rolB or rolA gene, optionally under the control of a heterologous promotor.

The transgenic plants of the invention can be used for breeding plants with altered biochemical, physiological and developmental physiological and/or morphological features.

Moreover, the transgenic plants, organs or tissue cultures derived from these plants and organs can be used for preparing secondary plant products, preferably alkaloids, monoterpenes, sesquiterpenes, diterpenes, triterpenesteroids, tetraterpenes, polyketides, polyacrylenes, flavonoids, phenylpropanoids, amines, alkaloids, non-protein amino acids, cyanoglycosides or glucosinolates.

The transgenic plants or organs of the invention can be prepared according to the following process.

First, protoplasts or tissues of a plant are cultured together with Agrobacterium tumefaciens bacteria or Agrobacterium rhizogenes bacteria which contain a recombinant plasmid derived from the Ti or Ri-plasmid and harbouring as inserts the coding regions of the $T_L$-DNA of the Ri-plasmid from Agrobacterium rhizogenes and/or the coding region of the IPT gene of the T-DNA of the Ti-plasmid from Agrobacterium tumefaciens and optionally a selectable marker gene. The only essential point in the preparation of the trans genic plant of the present invention is the use of specific recombinant plasmids.
Then transgenic cells are selected and transgenic plants or organs are regenerated from the transgenic cells.

In the preferred embodiments of the process of the invention, the recombinant plasmids are selected in such a way that the above-mentioned combinations of the coding regions can be inserted into the genome of the transgenic plant.

In accordance with the invention, those embodiments of the process for preparing the above-mentioned plants and organs of the invention are preferred, in which the recombinant plasmid contains coding regions and promotors having the biological- activity of, for instance, the plasmids pPCV002-ABC, pPCV002-AB, pPCV002-B1100, pPCV002-B300, pPCV002-CaMVBT, pPCV002-C, pPCV002-CaMVBT + C, pPCV002-CaMV-C, pPCV002-A, pPCV002-AC. The embodiments in which the recombinant plasmid contains coding regions and promotors with the biological activity as contained in plasmid pPCV002-BC or pPCV002-HSIPT are especially preferred.
Not only the specifically named plasmids, but also all biologically functional equivalents of these plasmids are suitable for the preparation of the transgenic plants and organs of the invention. The expert in this field who knows the invention is for instance able to isolate coding regions of rol genes from different Ri-plasmids occurring in Agrobacterium rhizogenes and to use them for constructing recombinant plasmids by which the coding regions can be integrated into the genome of desired plants to prepare transgenic plants and organs. Plasmid systems suitable for these purposes are for instance described in EP-A1 116 718. Similarly, it is possible according to the invention to use not only promotors which are identical in their DNA sequence, as for instance only a specific CaMV35S promotor, but also promotors which are equivalent in their biological function, as for instance 35S promotors which can be isolated from different strains of Cauliflower mosaic viruses.

The figures show:

Fig. 1: A schematique representation of the recombinant plasmid used.

Fig. 2: A comparison of a wild type-(SR1)-tobacco plant, right-hand side, a transgenic rolABC plant, lefthand side, and a transgenic rolAB plant, middle. The transgenic rolABC plant shows all phenotypical features of the hr syndrome, while the transgenic rolAB plant differs both from the wild type-(SR1) plant and from the rolABC plant.

Fig. 3: A comparison of a wild type tobacco plant (SR1) left-hand side, and a transgenic CaMV-C plant. The CaMV-C plant has a reduced apical dominance causing a dwarf growth habit. It has a higher number of side shoots and light green lanceolate leaves. The inflorescences have smaller flowers which are male sterile.

Fig. 4: Transgenic CaMV-C plants.

Fig. 5: In vitro root cultures.
The figure shows a comparison of the root growth of a wild type-(SR1) tobacco plant, rigth-hand side, and that of a transgenic CaMV-C plant cultivated in the dark in a hormone free, liquid medium (MS) for three weeks.

Fig. 6: Transgenic rolA plant.
The depicted transgenic rolA plant was prepared with the recombinant plasmid pPCV002-A. It has wrinkled leaves and a condensed inflorescence. This morphology is attributable to the expression of the coding region of the rolA gene.

Fig. 7: Comparison of a transgenic rolC plant, middle, a wild type-(SR1) plant, left-hand side, and a CaMV-C plant, right-hand side.
The transgenic rolC plant was constructed with the recombinant plasmid pPCV002-C. The transgenic rolC plant has slightly reduced apical dominance and smaller flowers with reduced pollen production. This phenotype is attributable to the expression of the coding region of the rolC gene under the control of the own promotor.

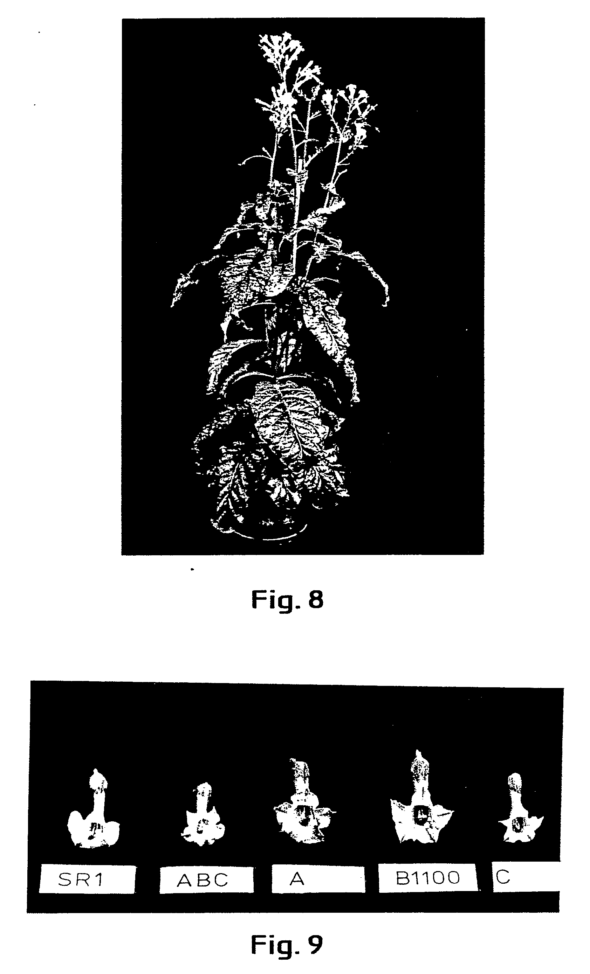

Fig. 8: Transgenic rolAC plant.
The depicted transgenic rolAC plant was prepared with the recombinant plasmid pPCV002-AC. It has wrinkled leaves, slightly reduced apical dominance and smaller flowers. This morphology is attributable to the combined expression of the coding regions of the rolA gene and the rolC gene.

Fig. 9: Flower morphology I.
The figure shows a comparison of the flower of the wild type-(SR1) plant and transgenic plants. Transgenic rolA and rolB plants have bigger flowers, while transgenic rolC plants have smaller flowers.

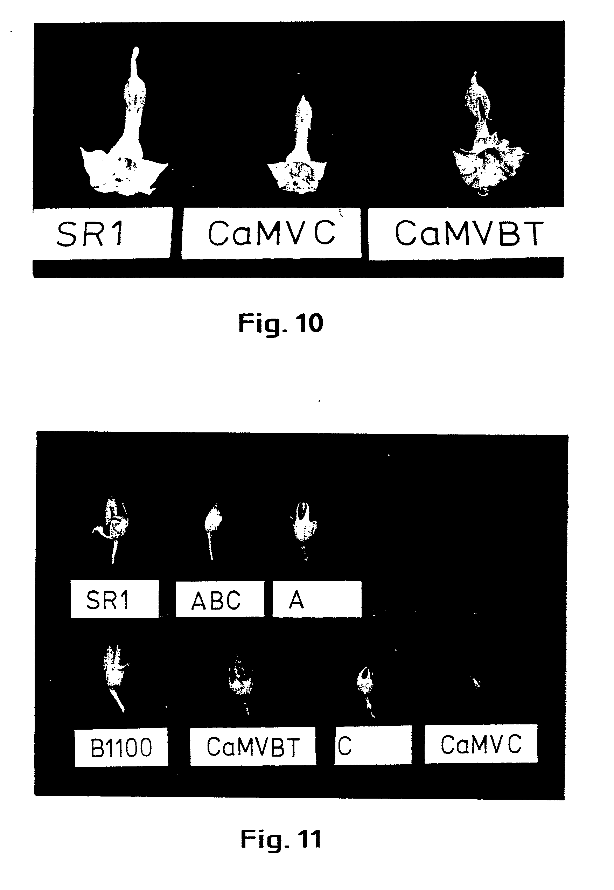

Fig. 10: Flower morphology II.
The figure shows a comparison of the flowers of wild type-(SR1) plants and transgenic plants. The flowers of transgenic CaMV-C plants are very small and male sterile. The flowers of transgenic CaMVBT plants in which the same promotor controls the expression of the coding region of the rolB gene have protruding stigmas and reduced stamen.

Fig. 11: Seed capsule size.
The figure shows a comparison of seed capsules of wild type (SR1) plants and seed capsules of transgenic plants. While the seed capsule size of transgenic rolA and rolB plants is unaltered, the seed capsules of transgenic rolC plants is distinctly smaller. The seed capsules of transgenic CaMV-C plants are even smaller. All depicted seed capsules were obtained by cross pollination of female recipients with SR1-pollen.

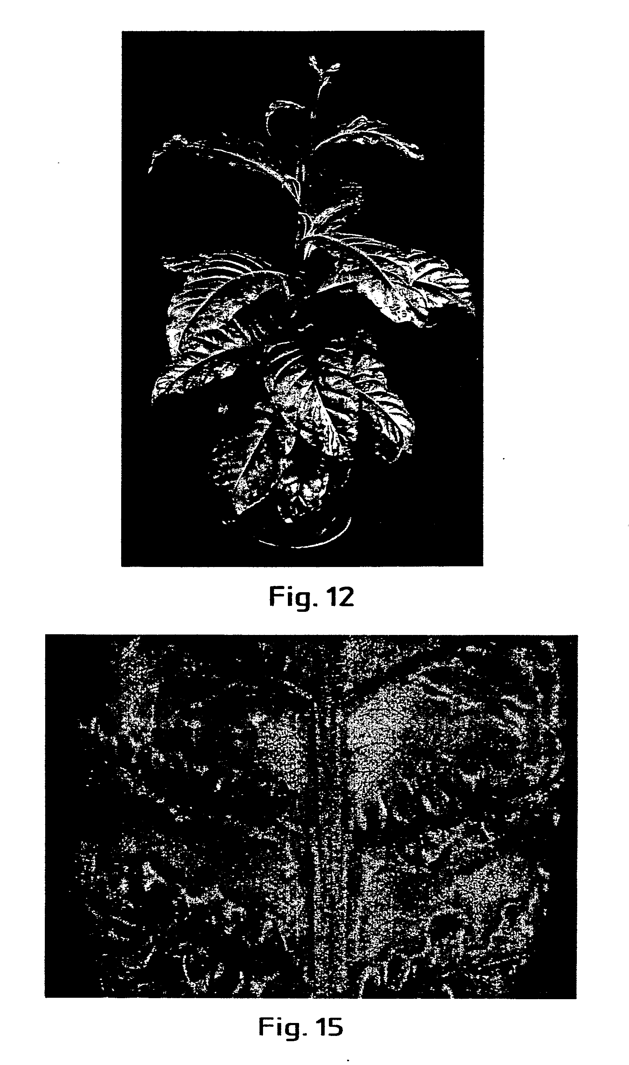

Fig. 12: Transgenic rolBC plant.
The depicted transgenic rolBC plant was obtained by means of the recombinant plasmid pPCV002-BC. It has a reduced apical dominance and smaller flowers. This morphology is attributable to the expression of the rolC gene. On account of the combined expression of the coding regions of the rolB and rolC gene, this transgenic plant has an increased root system.

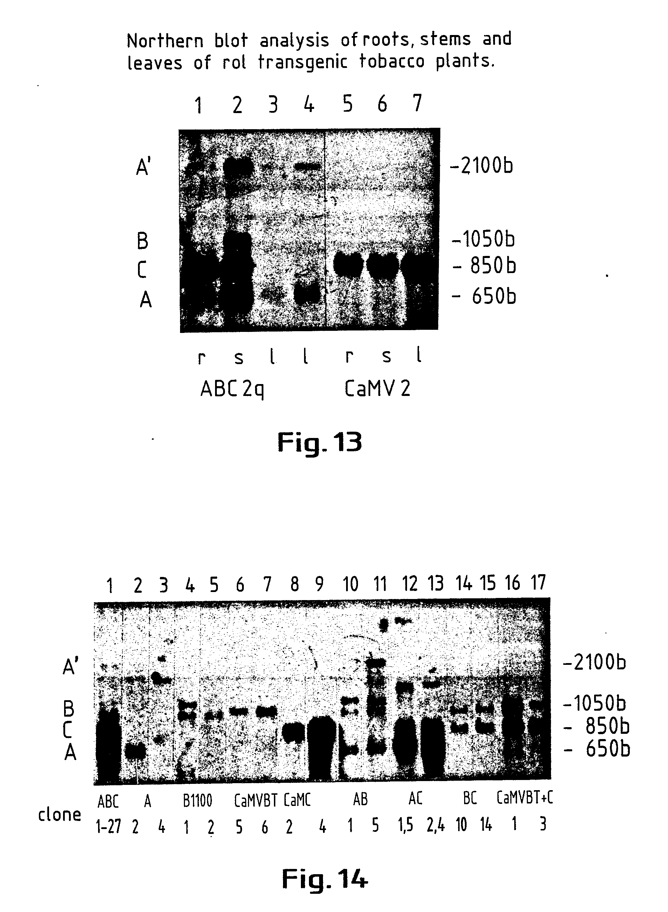

Fig. 13: Northern-Blot-Analysis of polyA-RNA from transgenic rolABC plants and polyA-RNA from transgenic CaMV-C plants.
The coding region of the rolC gene is expressed in transgenic rolABC plants in an organ specific fashion. By contrast, the coding region of the rolC gene is expressed in transgenic CaMV-C plants in a constitutive manner.
Lane 1-4: polyA-RNA of roots (r) stems (s) and leaves (1) of a transgenic rolABC plant.
Lane 5-7: polyA-RNA of roots (r), stems (s) and leaves (1) of a transgenic CaMV-C plant.
The Northern blot was hybridized with the radio active labelled EcoRI fragment 15 of the $T_L$-DNA of Agrobacterium rhizogenes, which contains the rolA, B and C genes.

Fig. 14: Northern blot analysis of polyA-RNA from various transgenic plant tissues. The depicted Northern blot shows the expression of the different rol genes. polyA-RNA of the following transgenic plant tissues was applied:

Lane 1: clone rolABC 1-27
Lane 2: clone rolA-2
Lane 3: clone rolA-4
Lane 4: clone rolB1100-1
Lane 5: clone rolB1100-2
Lane 6: clone ro1CaMVBT-5
Lane 7: clone rolCaMVBT-6
Lane 8: clone rolCaMV-C-2
Lane 9: clone rolCaMV-C-4
Lane 10: clone rolAB-1
Lane 11: clone rolAB-5
Lane 12: clone rolAC-1,5
Lane 13: clone rolAC-2,4
Lane 14: clone rolBC-10
Lane 15: clone rolBC-14
Lane 16: clone rolCaMVBT + C1
Lane 17: clone rolCaMVBT + C3

Fig. 15: The figure represents leaf necrosis of a transgenic CaMVBT plant, which are caused by the higher expression of the rolB gene in leaves due to its specific expression under the control of the CaMV35S promotor.

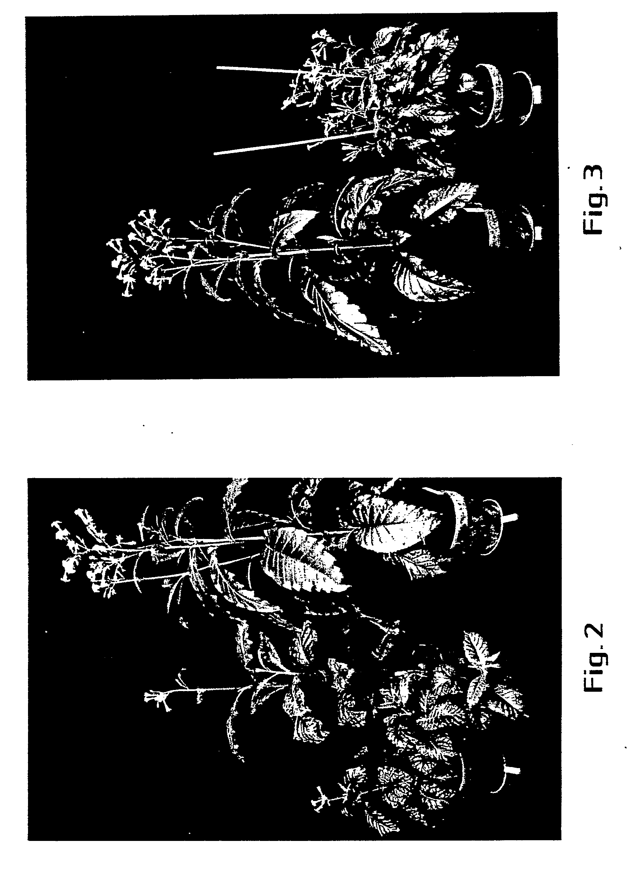

The fully developed hr syndrome is found in transgenic plants which express the rolA, rolB and rolC gene; see Spena et al., loc cit. The analysis in accordance with the inven tion of transgenic plants which contain in their genome different combinations of the coding regions of these three rol genes revealed distinguishable and specific growth abnormalities which correlate with the expression of the mentioned coding regions; see figures 2, 6 to 9, 11, 12. Transgenic plants which contain only the coding region of one single rol gene in their genome do not show all phenotypical features of the hr syndrome. Hence, the hr syndrome is produced by a combined effect of genes, each of which has a distinguishable biological effect in the transgenic plants.

It was already known that the rolB and rolC genes in Ri transgenic tobacco plants and in Ri transgenic potato plants, which however all contain at least three genes, i.e. the rolA, rolB and rolC gene in their genome, are expressed in an organ specific way. Moreover, an organ specific expression from transgenic tobacco plants is known which contain all three genes; see Spena et al., loc. cit. In these experiments it was shown by Northern blot analysis that the expression rate of the rolB and rolC gene is high in the stems, somewhat lower in the roots and much lower in the leaves. These results have been confirmed by the analysis of the expression rate of a reporter gene under the control of the rolB and rolC promotor. It was also possible to determine the organ specificity and cell specificity of the expression more precisely.

Modifications of the expression rate also give rise to developmental alterations. The increased number of side shoots, for instance correlates with a higher expression rate of the rolC gene (figure 13). The specificity of the expression rate plays an important role in the genesis of morphological alterations of transgenic hr plants. Transgenic plants which contain chimaeric genes in which the coding region of the rolB or rolC gene is under the control of a CaMV35S promotor show new morphological growth abnormalities. With the aid of the Northern blot analysis and histochemical stainings it has been shown that the leaf necrosis correlates with an increased expression of the rolB gene in the leaves (figure 15). The increased expression of this gene can be achieved by expression under the control of a CaMV35S promotor.

The distinguishable growth patterns of transgenic tobacco plants which contain in their genome chimaeric CaMVBT and CaMV-C genes in which the two coding regions are controlled by the same promotor suggest that the biological effect of these gene products on the entire plant is different. Hence the rolB and rolC gene products either have different biochemical targets or have a different effect on the same target. The modifications caused by the expression of the rol genes are indicative of modifications of hormone controlled processes. Therefore, models have been proposed in which the hr syndrome is explained by increased auxin sensitivity caused by a rol induced modification of the hormone signal reception-transduction system. On the other hand, it has been assumed that a function of the rol gene leads to modifications in the cytokinin biosynthesis or has a cytokinin-like effect. In the corresponding experiments, the virulence of Ti-plasmids mutated in the region of the IPT gene was restored by the incorporation of a region of the $T_L$-DNA of the Ri-plasmid. The biological effects of the products of rolA and rolB genes does in fact remind of effects caused by auxin, and the biological effects prompted by the rolC gene suggests a cytokinin activity. Nevertheless, the effects of the rol genes are not easily explained by models which require the synthesis of transported phytohormones, such as indole acetic acid (IAA) and isopentenyladenosine (IPT). The effect of these genes is limited to the transformed cells at least in root formation. rolB and/or rolA induced auxin-like effects could be obtained not only by inducing an alteration of the reception-transduction system for the auxin signal, but also by inhibiting the effect of auxin antagonists, such as cytokinins, or by synthesizing a substance having an auxin-like effect, such as phenyl acetic acid, which is not transported in the plant tissues. In both cases, the result would be an increased biological auxin effect without alteration of the IAA concentration. On the other hand, the features found in transgenic CaMV-C plants and the effects produced in hr plants and transgenic CaMV-BT + C plants by an increased expression rate of the rolC gene are typical of increased cytokinin activity. Accordingly, the increased cytokinin activity may also be attributable to the inhibition or partial inactivation of indole acetic acid or to the synthesis of a cell autonomous substance with cytokinin activity.

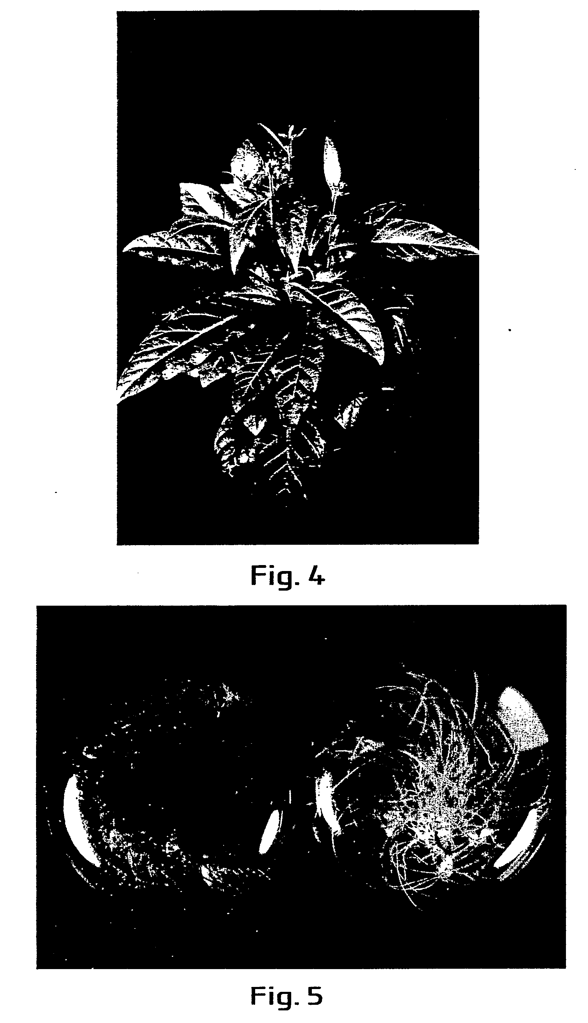

The examination of the growth behaviour of transgenic tobacco root cultures revealed that root growth can be substantially improved in in-vitro root cultures by the combination of the rolB and rolC gene in particular, and somewhat less efficiently by the combination of the rolA and rolC gene. Here, the rolB gene of the group consisting of the rolA, rolB and rolC genes is the most efficient one in inducing root formation. However, rapid growth of branched roots is better achieved by the rolC gene. The product of the rolC gene has a rather limited capability of inducing root formation. Transgenic rolC and in particular CaMV-C roots are highly branched and show rapid and plagiotropic growth, see figure 5.

It is known that auxins induce the formation of additional roots (rhizogenesis). However other phytohormones, such as cytokinins, also have an influence on rhizogenesis. Moreover, there exist indications suggesting that a high auxin/cytokinin ratio is optimal for inducing root formation, while a low auxin/cytokinin ratio is necessary for the root growth to continue. Consequently, the combined effect of the rolB and rolC gene products is advantageous for effective root formation and effective root growth; the rolB gene product (auxin-like effect) effectively induces the root formation and the rolC gene product (cytokinin-like effect) permits rapid and branched root growth by counteracting the biological activity of the rolB gene product.

The recombinant plasmids used in the following examples, except for the plasmid pPCV002-BC, are described in Spena et al., loc. cit.

The transgenic plants and organs of the present invention can be prepared for instance by transforming protoplasts by means of the recombinant plasmids, subsequently selecting transgenic cells and regenerating whole plants from the selected transgenic cells.

Material and Methods

Bacterial strains and cultures

The bacterial strains and cultures are described in Spena et al., loc. cit. All recombinant plasmids were first transferred into E. coli, strain SM10, and then mobilized in Agrobacterium tumefaciens, strain GV3101. Koncz and Schell, Mol. Gen. Genet. 204 (1986), p. 383.

Construction of recombinant plasmids.

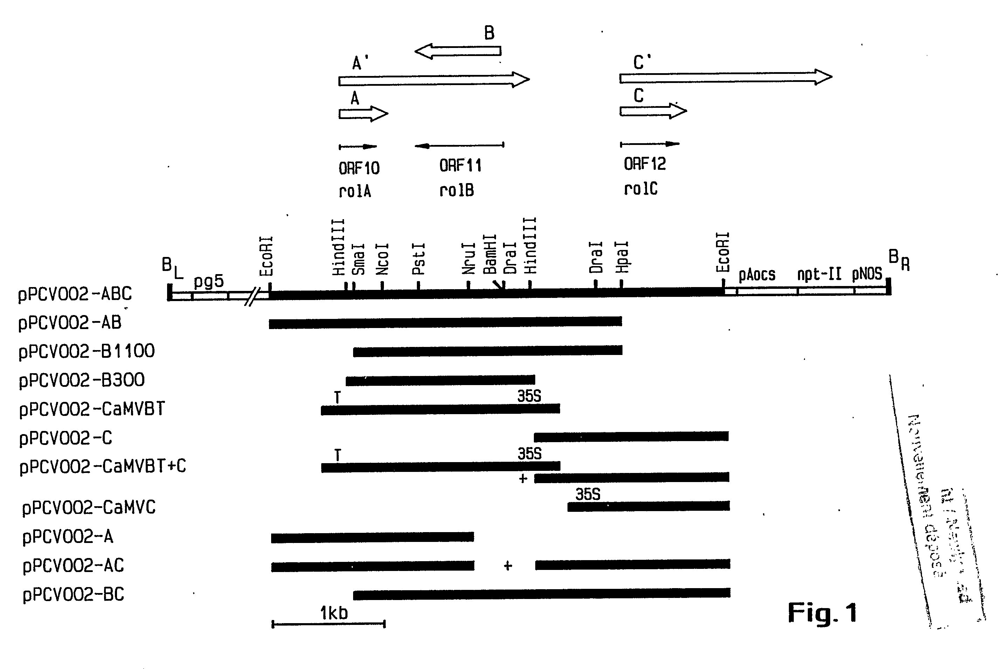

The used recombinant plasmids were constructed according to established methods; Maniatis et al., "Molecular Cloning, A Laboratory Manual". Cold Spring Harbour Laboratory, 1982. The plasmids shown in figure 1 have already been described in Spena et al., loc. cit, except for the recombinant pPCV002-BC plasmid.

Plant tissue cultures and transformation

The root induction on leaves of Kalanchoe diagremontiana and on leaf disks of sterile shoot cultures of Nicotiana tabacum cv. Petit Havana SR1 (Nagy and Maliga, Z. Pflanzenphys. 78 (1976), p. 453) was performed as described by Spena et al., loc. cit.

In order to examine whether the rol gene induced roots were transformed in all cases, leaf disks were incubated with different constructs and cultured on MS medium without kanamycin; Murashige and Skoog, Physiol. Plant. 15 (1962), p. 473. After four weeks the roots were cut and divided into two halves. One half was subjected to kanamycin selection (50 mg/l) on MS medium during callus formation. The MS medium was supplemented with 0.6 mg/l of naphthyl acetic acid (NAA) and 0.2 mg/l kinetin. Transgenic plants were regenerated in the usual manner from transgenic calli on an MS medium supplemented with 0.5 mg/l of benzylaminopurin (BAP) and 0.1 mg/l of NAA. The regeneration of transgenic plants from calli, however, poses difficulties, if the cellular genome incorporates the coding regions derived from recombinant plasmids pPCV002-CaMVBT or pPCV002-CaMVBT + C . Therefore, the shoots were cultured for 10 to 12 weeks on MS medium supplemented with 7.5 mg/l of isopentenyl adenosine and 0.1 mg/l of chlorophenoxy acetic acid; Firoozabady, Plant Science 46 (1986), p. 127. The shoots were rooted on hormone free MS medium and selected for kanamycin resistance. The root cultures were established either on solid or liquid MS medium.

DNA and RNA analysis

DNA and RNA were obtained from the tissues of the transgenic plant according to Taylor and Powell; BRL Focus 4 (1983), p. 4. The polyA-RNA was purified by chromatography on oligo-dT-cellulose in accordance with the instructions of the manufacturer (Boehringer Mannheim) and then separated on a 1.5% agarose formaldehyde gel. They were then transferred to nylon membranes and hybridized with radioactive probes. The probes used were purified DNA fragments labelled by nick translation with a nick translation kit of BRL in accordance with the instructions of the supplier. The hybridization of the Northern blot was performed in 10% dextran sulfate, 1M sodium chloride, 1% sodium lauryl sulfate, 100 $\mu$g/ml of heterologous DNA at 65° C. Washing was performed first with 2xSSPE, 1% SDS at room temperature and subsequently with 0.2xSSPE and 1% SDS at 65° C. The DNA blots were then further treated in the usual manner; Maniatis et al. loc. cit.

## Proof of neomycinphosphotransferase II activity

Neomycinphosphotransferase II activity was proved by in-situ tests as described by Spena et al., EMBO J. 4 (1985), p. 739.

The examples illustrate the invention.

## Example 1

### Construction of the plasmid pPCV002-BC

The recombinant plasmid pPCV002-BC was obtained by subcloning the 3581bp-SmaI/EcoRI-DNA fragment in the binary vector pPCV002. A precise description of the fragment is given in Spena et al., loc. cit. A SmaI/EcoRI-fragment containing the coding regions of the rolB and rolC genes was isolated from the plasmid pPCV002-ABC and subcloned into the SmaI/EcoRI-restriction cleavage sites of the plasmid pUC19. This fragment spans the region from the base pair 9863 to 13,445 according to the numbering of the $T_L$-DNA sequence by Slightom et al. (1986), loc. cit. From the plasmid pUC19-BC thus obtained, an EcoRI/XbaI-DNA fragment was cut out and subcloned into the EcoRI/XbaI-restriction cleavage sites of the vector pPCV002. In this way, the plasmid pPCV002-BC was obtained.

## Example 2

### Construction of the plasmid pPCV002-HSIPT

The plasmid pPCV002-HSIPT was constructed according to established methods; see Maniatis et al. (1982), loc. cit. A RsaI/RsaI-DNA fragment from the T-DNA of the octopine Ti- plasmid $pT_i15955$ was subcloned into the SmaI-restriction cleavage site of the plasmid pUC9. This DNA fragment spans the region from base pair 8488 to 9836 in accordance with the numbering by Barker et al., Plant Mol. Biol. 2 (1983), pp. 335 to 350. The plasmid pUC9-IPT obtained was cut by the restriction enzyme EcoRI at a restriction cleavage site upstream the coding region of the IPT gene and the promotor was then removed by digestion with exonuclease Ba131. The number of the base pairs remaining upstream the translation start site was checked by DNA sequencing in accordance with the chain termination method. The coding region of the IPT gene was then removed from the plasmid pUC9-IPT by restriction cleavage by the enzymes EcoRI and HindIII and subcloned into the BamHI/HindIII restriction cleavage sites of the plasmid pUC8-HSP-70, the EcoRI and the BamHI restriction cleavage sites having been converted into blunt ends by being treated with Klenow polymerase prior to ligation. The resulting plasmid pUC8-HSIPT thus contains the coding region of the IPT gene downstream the heat-inducible HSP-70 promotor of Drosophila melanogaster. This chimaericgene was cut out by the restriction enzymes EcoRI and HindIII and subcloned into the EcoRI and HindIII restriction cleavage site of the binary plant vector pPCV002. Consequently, the plasmid pPCV002-HSIPT obtained possesses the following structural features:
- 258bp of the HSP-70 promotor of Drosophila melanogaster;
- 199bp untranslated sequence of Drosophila melanogaster;
- 16bp untranslated sequence of the IPT gene with the nucleotide sequence "GGATCTAATTC" at the fusion site;
- 712bp coding region of the IPT gene; and - 373bp 3'-untranslated sequence of the termination region of the IPT gene.

## Example 3

### The biological effect of the rolA, rolB and rolC gene in transgenic tobacco plants

Transgenic tobacco plants in which the rolA, rolB and the rolC genes are expressed show all the phenotypical alterations characteristic of the hr syndrome. By contrast, transgenic tobacco plants in which a single rol gene or double combinations of these genes are expressed show other phenotypical alterations characteristic of such single genes or combinations. In figure 2 a transgenic tobacco plant which contains and expresses the coding regions of the rolA and the rolB gene is compared with a normal wild type-(SR1) tobacco plant or a transgenic tobacco plant which contains and expresses the coding regions of the rolA, rolB and rolC genes. It is easily recognizable that the plant of the invention which contains and expresses the coding regions of the rolA and the rolB gene differs both from the wild type plant (SR1) and from the hr syndrome plant which expresses all three coding regions.

Transgenic plants which contain combinations of the coding regions of the rolB and the rolC gene or the rolA and the rolC gene in their genomes do not show all features characteristic of the hr syndrome, but differ from wild type plants; see figure 12 and figure 8, respectively.
The same conclusions are drawn when examining the progeny of cross experiments in which transgenic rolAB plants are crossed with transgenic rolC plants. In these cross experiments performed in a known manner a transgenic tobacco plant which contains in its genome the coding region of the rolA and the rolB gene (for instance AB3-5) is crossed with a transgenic tobacco plant which contains the coding region of the rolC gene in its genome (for instance C9a). 29 of 100 plants of the offspring showed the complete hr syndrome.

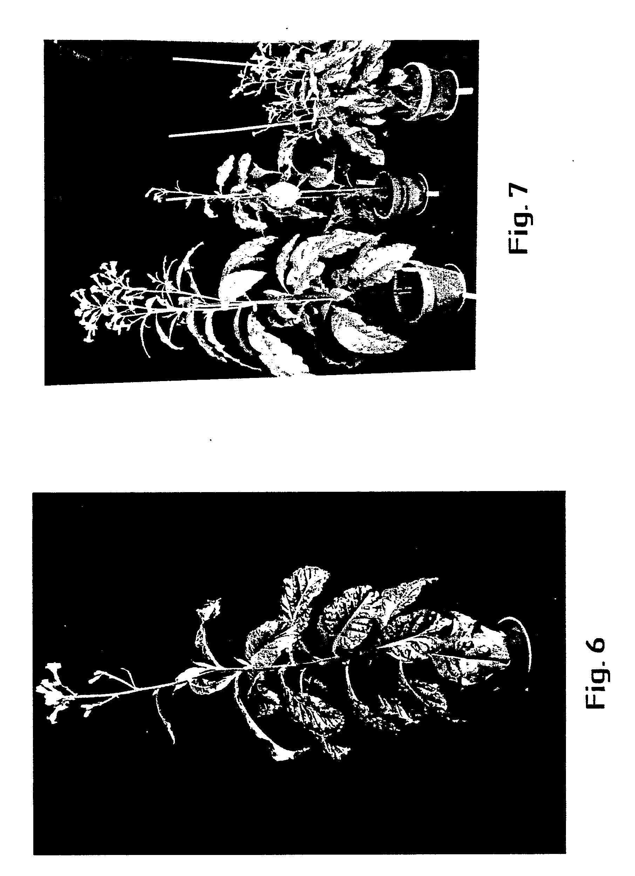

Transgenic tobacco plants which contain and express the coding region of the rolC gene, show a modified leaf morphology, reduced flower size and pollen production (figure 9), the seed capsules are smaller (figure 11) and the plant is more branched (figure 7).

Transgenic tobacco plants which contain the coding region of the rolB gene in their genome and express it, all show other growth alterations. These are essentially other alterations of leaf morphology, enlarged stigmas and flowers (figure 9). Pollen production is only slightly reduced and the seed capsules are not adversely affected (figure 11). Flowers of tobacco plants, transgenic for the rolA, rolB and rolC genes, are smaller than those of wild type plants (SR1), while flowers of plants transgenic for the rolB gene are larger and show altered features (figure 9).

Transgenic plants which contain in their genome a combination of the coding region of the rolC gene and the coding region of the rolA gene or rolB gene, also have smaller flowers. In these plants, one of the coding regions is optionally under the control of a strong heterologous promotor. Such plants can, for instance, be obtained by means of the plasmids pPCV002-AC, pPCV002-BC or pPCV002-CaMVBT + C.

Transgenic tobacco plants which contain the coding region of the rolA gene in their genome differ in their modified growth properties both from transgenic plants which contain the coding region of the rolB gene in their genome and from those which contain the coding region of the rolC gene in their genome. An especially typical feature of such plants are wrinkled leaves, condensed inflorescences (figure 6) and larger flowers with modified morphology (figure 9).

The progeny of the transgenic plants of the invention show morphological alterations similar to those of the parent plant. Moreover, in the experiments performed, it was found that the altered features are cosegregating with the resistance to kanamycin sulfate. In another experiment, various tobacco plants according to the invention were tested to examine whether the phenotypical alterations correlate with the expression of rol genes.
Figure 13 shows a Northern blot analysis of polyA-RNA extracted from the tissue of various transgenic plants of the invention. In all cases tested, the altered growth correlates with the expression of rol genes.

Example 4

Alteration of the rol typical features by modification of the expression rate

Transgenic plants which contain in their genome the coding region of the rolC gene which is under the control of the CaMV35S promotor have a phenotype different from that of plants in which the coding region of the rolC genes contained in the genome is under the control of the natural promotor; see Figures 3, 4 and 7. The use of the CaMV35S promotor results in a bushy phenotype with an increased number of shoots. The flowers are very small (figure 10) and the leaves are narrow and lanceolate (figure 4). The transgenic CaMV-C plants are male sterile, and the trait is transmitted to its progeny as a single dominant gene.

Tests in a system with transient expression using tobacco leaf protoplasts have shown that the expression rate of the coding region of the rolC gene is 15 times higher when the CaMV35S promotor is used. This matches with the results of the Northern blot analyses performed with polyA-RNA from transgenic plants. This is also confirmed by the analysis of the expression in transgenic plants containing a chimaeric gene consisting of the coding region of the β-glucuronidase and the rolC promotor. Consequently, the modification of the specific expression of the coding region of the rolC gene is responsible for the reduction in apical dominance.

Moreover, transgenic plants were prepared in which the coding region of the rolB gene is under the control of the CaMV35S promotor. These plants were prepared using the recombinant plasmid pPCV002-CaMVBT. One of the features of these transgenic plants is an earlier leaf senescence (necrosis); see figure 15. The Northern blot analysis of the polyA-RNA from these transgenic plants showed that the chimaeric gene is expressed in leaves to a higher degree than in transgenic plants in which the coding region of the rolB gene is under the control of the natural promotor. The new phenotype is thus caused by the increased expression rate of the coding region of the rolB gene.

Furthermore, transgenic plants in accordance with the invention were prepared which contain a combination of the coding region of the rolB gene under the control of the CaMV35S promotor and the coding region of the rolC gene. The recombinant plasmid pPCV002-CaMVBT + C was used for preparing these plants. Said plants show a leaf necrosis typical of transgenic CaMVBT plants; see figure 15. By contrast, the flower morphology is comparable to transgenic plants which contain the coding region of the rolC gene in their genome.

The necrotic processes start in the leaf tissue between the veins and proceed to affect the whole leaf; see figure 15. This is usually observed in plants which have reached maturity before flower formation. The leaves first affected are neither the oldest nor the youngest but are situated approximately in the middle of the plant. However, some transgenic CaMVBT + C plants have an increased number of shoots and leaves, with the necrotic process being less pronounced in these plants and shifted to the period after blooming. Northern blot analysis of different transgenic CaMVBT + C plants suggests that the plants with an increased number of shoots and delayed leaf necrosis express the CaMVBT gene less than the rolC gene. Thus these plants have an increased ratio of rolC transcripts to rolB transcripts.

## Example 5

### Transgenic, rol gene induced tobacco roots

In the first place it was examined whether rol gene induced tobacco roots are able to grow on MS medium containing kanamycin, as the roots, because of their construction by the plasmids pPCV002-ABC, pPCV002-B1100, pPCV002-AC, also contain the neomycinphosphotransferase II gene, apart from the coding regions of the rol genes. 166 rol gene induced tobacco roots were tested. 153 of them were clearly able to grow on the MS medium containing kanamycinsulfate. Other 12 were not distinctly positive and were therefore examined in in-situ tests for the activity of the neomycinphosphotransferase II gene. In addition, Southern blots of the plant genome were prepared. The results show that these roots have in fact been transformed.

The different coding regions of the rol genes or their combinations have different effects on root growth. Transgenic tobacco roots containing the individual rol genes grew better than untransformed roots. Transgenic roots containing the rolC gene branched more often than those containing the rolA gene or the rolB gene. By contrast, transgenic roots containing combinations of the rolC gene with the rolA gene and or the rolB gene grew more vigorously. The rolB gene when combined with the rolC gene has a stronger effect than the combination of the rolA gene with the rolC gene. Combinations of the gene rolA and rolB did not result in substantially enhanced root growth as compared to the genes used alone. When the coding region of the rolC gene is under the control of the CaMV35S promotor, root growth is enhanced (figure 5). By contrast, the roots grow less vigorously when the expression of the rolB gene is under the control of the CaMV35S promotor. However, roots grow faster if the coding region of the rolB gene is under control of the CaMV35S promotor and is combined with the rolC gene. In this case, the roots are highly branched and grow in a plagiotropic manner. The recombinant plasmids described in Spena et al., loc. cit. and in example 1 were used to prepare these roots derived from transgenic plants.

Tissue cultures of the roots are particularly suitable for preparing secondary plant products.

## Claims

1. A transgenic plant or plant organ with modified physiology, morphology and modified hormone metabolism which contains in its genome a) the coding region of the rolA gene, rolB gene, rolC gene, rolA and rolC gene, rolB gene and rolC gene or rolA and rolB gene of the $T_L$-DNA of the Ri-plasmid from Agrobacterium rhizogenes and/or the coding region of the IPT (isopentenyladenosine transferase) gene of the T-DNA of Ti-plasmids from Agrobacterium tumefaciens, said coding region(s) being under the control of its/their natural or a heterologous promotor(s), except for the coding region of the rolC gene, which, if it is not contained in combination with one of the other coding regions in the genome, is under the control of a heterologous promotor; and

b) optionally also a selectable marker gene;

wherein the coding regions and the marker gene are expressed.

2. The transgenic plant or plant organ according to claim 1 in which the heterologous promotor is the HSP ("Heat Shock Promotor")-70-promotor of Drosophila melanogaster.

3. The transgenic plant or plant organ according to claim 1 or 2, in which the heterologous promotor is a strong constitutive promotor.

4. The transgenic plant or plant organ according to claim 1 or 3, in which the strong constitutive promotor is the CaMV ("Cauliflower Mosaic Virus")-35S-promotor.

5. The transgenic plant or plant organ according to one of claims I, 3 or 4 which contains in its genome the coding region of the rolC-gene of the $T_L$-DNA of the Ri-plasmid from Agrobacterium rhizogenes which is under the control of a strong constitutive promotor, preferably the CaMV35S-promotor.

6. The transgenic plant or plant organ according to claims 1 to 5, in which the coding region of the IPT gene is under the control of the HSP-70 promotor of Drosophila melanogaster.

7. The transgenic plant or plant organ according to claims 1 to 6, which contains in its genome the coding region of the rolB gene and the rolC gene under the control of a roots-specific promotor and thus shows a modified root formation and modified root growth in the absence of the other features typical of transgenic rolBC plants.

8. A transgenic plant or plant organ with modified physiology, morphology and modified hormone metabolism, which contains in its genome the coding region of the rolA, rolB and rolC gene, with at least one of the coding regions being under the control of a heterologous promotor, preferably the CaMV35S promotor, and the other coding regions being under the control of their natural promotor(s).

9. The transgenic plant or plant organ according to any one of claims 1 to 8, which is derived from solanaceae, apocynaceae, chenopodiaceae, polygonaceae, boraginaceae, compositae, rubiaceae, scrophulariaceae, caprifoliaceae, leguminosae, araceae, moraceae, asplenium, euphorbia ceae, cabbage types, sunflowers, carrots, paprika (Capsicum annuum), leek, raphanus types, lettuce types, celery (ApiuM graveolens), onions, fennel (Foeniculum vulgare), wheat, rye, barley, corn (maize), sugar beet, soybean, oats, rice types, dahlias, ornamental tobacco, pelargonia, petunia, primulae, violets, asters, amaranthus types, windflowers, lionsmouths (Antirrhinum), aquilegia, ornamental asparagus, begonias, calceolaria, coleus, chrysanthema, cyclamen, delphinium, carnations, gerbera, impatiens, lupine, gilliflower, portulaceae, crowflower (Ranunculus), sage, gloxinia, tagetes, mullein, zinnia, rosaceae, saponoria, or Panax ginseng, preferably from potato or tobacco.

10. A tissue culture containing cells or organs of a transgenic plant or cells of a transgenic plant organ according to anyone of claims 1 to 9.

11. The use of a transgenic plant according to any one of claims 1 to 9 for breeding plants with modified biochemical, physiological, developmental physiological and/or morphological features.

12. The use of a transgenic plant according to any one of the claims 1 to 9 or a tissue culture according to claim 10 for preparing secondary plant products, preferably alkaloids, monoterpenes, sesquiterpenes, diterpenes, triterpenesteroids, tetraterpenes, polyketides, polyacetylenes, flavonoids, phenylpropanoids, amines, non-protein amino acids, cyanglycosides or glucosinolates.

13. A process for preparing a transgenic plant or plant organ according to any one of claims 1 to 9, wherein

a) protoplasts or tissue of a plant are cultured together with Agrobacterium tumefaciens or Agrobacterium rhizogenes bacteria containing a recombinant plasmid which is derived from Ti or Ri-plasmids and which contains the coding region(s) of the $T_L$-DNA of the $R_i$-plasmid from Agrobacterium rhizogenes and/or the coding region of the IPT gene of the T-DNA of the Ti-plasmid from Agrobacterium tumefaciens and optionally a selectable marker gene;

b) transgenic cells are selected; and

c) a transgenic plant or a transgenic plant organ is regenerated from the transgenic cells.

14. The process according to claim 13 wherein the recombinant plasmid contains coding regions and promotors with the biological activity of the coding regions and promotors contained in the recombinant plasmids pPCV002-ABC, pPCV002-AB, pPCV002-B1100, pPCV002-B300, pPCV002CaMVBT, pPCV002-C, pPCV002-CaMVBT+C, pPCV002-CaMV-C, pPCV002-A, pPCV002-AC, pPCV002-BC or pPCV002-HSIPT.

15. A recombinant plasmid which contains coding regions or promotors with the biological activity of the coding regions and promotors contained in the recombinant plasmid pPCV002-BC.

16. A recombinant plasmid which contains coding regions and promotors with the biological activity of the coding regions and promotors contained in the recombinant plasmid pPCV002-HSIPT.

17. A process for eliminating male sterility or reduced female fertility of a transgenic plant according to any one of claims 1 to 9, wherein the transgenic plant is cross-bred with a male fertile plant, which is homozygous for a gene which

a) under the expression control of a promotor functional in the transgenic plant expresses an antisense-RNA, which forms a hybrid with the mRNA of the gene causing male sterility or reduced female fertility; or

b) under the expression control of a promotor functional in the transgenic plant expresses a ribozyme which recognizes and cleaves in the offspring the mRNA of the gene causing male sterility or reduced female fertility.

Fig. 1

Fig. 3

Fig. 2

Fig. 4

Fig. 5

Fig. 7

Fig. 6

**Fig. 8**

**Fig. 9**

**Fig. 10**

**Fig. 11**

**Fig. 12**

**Fig. 15**

Northern blot analysis of roots, stems and leaves of rol transgenic tobacco plants.

Fig. 13

Fig. 14